# EUROPEAN PATENT SPECIFICATION

(11) **EP 2 797 564 B1**
(45) Date of publication and mention of the grant of the patent: **09.09.2015**
(21) Application number: 12805904.5
(22) Date of filing: 18.12.2012
(51) Int. Cl.: A61F 13/15, A61F 13/511

(54) **PROCESS FOR MAKING ABSORBENT ARTICLES**
VERFAHREN ZUR HERSTELLUNG SAUGFÄHIGER ARTIKEL
PROCÉDÉ DE FABRICATION D'ARTICLES ABSORBANTS

(30) Priority: 29.12.2011 US 201161581118 P
(43) Date of publication of application: 05.11.2014
(73) Proprietor: The Procter & Gamble Company, Cincinnati, OH 45202 (US)
(72) Inventor: ERGUEN, Tamer, D-74564 Crailsheim (DE); WEISE, Martin, CH-1213 Petit-Lancy (CH)
(74) Representative: Briatore, Andrea
(86) International application number: PCT/US2012/070276
(87) International publication number: WO 2013/101540

(56) References cited:
- EP-A1- 1 842 512

## Description

### FIELD OF THE INVENTION

The present invention relates to a continuous process for making absorbent articles, in particular feminine hygiene articles, wherein different web materials are provided in a converting line and assembled. In the process of the invention, a nonwoven web is provided and is continuously slit to form two sub-webs that are used as different components of the absorbent articles.

### BACKGROUND OF THE INVENTION

Absorbent articles such as pantiliners, sanitary napkins or diapers are made on converting lines functioning at high speed. The different materials forming the absorbent articles are usually supplied as rolls that are continuously unwound and converted to form the various layers of the absorbent article. Such layers normally include at least a topsheet, an absorbent core and a backsheet, and commonly intermediate or additional layers such as a secondary topsheet, secondary backsheet, a layer of adhesive placed on the backsheet to adhere the articles within the undergarment, and the like.

The topsheet is the uppermost layer of the article and is placed in contact with the wearer's skin when the article is worn. Various topsheet constructions have been proposed. A low cost topsheet may be for example a light weight non-woven layer but this simple material has relatively poor absorbency and rewet properties. Formed films are also commonly used and have usually better fluid management properties but are more costly and may give a poor feeling compared to softer non-woven materials. Hybrid topsheets have been proposed, wherein a formed film is complemented with two lateral nonwoven topsheet stripes placed above it, as described, for example, in WO 2007/116347 A2.

Composite topsheets have also been proposed, in which a formed film and a nonwoven layer are attached to combine the softness of a nonwoven film and the fluid management properties of a formed film, as described, for example, in US Statutory Invention Reg. No. H1670.

EP 1 842 512 A1 discloses a method for forming an absorbent article having first and secondary topsheets.

Despite these various improvements, there remains a need for reducing the cost of production of these articles while maintaining the speed of manufacturing and the quality of the product. Using lower cost materials for one or the other layer usually means sacrificing on some of the material's attributes such as absorbency capacity, speed of absorbency or fluid retention.

### SUMMARY OF THE INVENTION

The present invention relates to a continuous process for making an absorbent article in a converting line, such as diapers or feminine hygiene products including sanitary napkins and pantiliners. The process comprises the step of providing a nonwoven web, an apertured formed film, a backsheet material, and an absorbent core material. The process further comprises the steps of:
- slitting the nonwoven web along its length to form a first sub-web and a second sub-web of approximately equal width;
- superposing the first sub-web, the apertured formed film, the second sub-web, the absorbent core material and the backsheet material in that order or reverse order; and
- forming the absorbent article by attaching and cutting the superposed first sub-web, the apertured formed film, the second sub-web, the absorbent core material and the backsheet material.

The first sub-web can be attached to the apertured formed film material to form a composite topsheet material before being attached to the other materials. The first sub-web may for example be glued to the apertured formed film.

The layers indicated in the preamble should not be considered to be an exhaustive list. Additional layers may of course be present below, between or above any of the layers indicated. For example, lateral topsheet stripes may be further placed on the outward surface of the first sub-web or a secondary backsheet material may be further placed between the absorbent core and the backsheet material.

### BRIEF DESCRIPTION OF THE DRAWINGS

FIG. 1 is a schematic perspective view of a slitting device used to slit the nonwoven web along its length to form a fist sub-web and a second sub-web.
FIG. 2 is a schematic perspective view of the slitting device of FIG. 1 from a different angle.
FIG. 3 is a diagram showing a converting process according to the invention.
FIG. 4 shows a cross-section of an exemplary absorbent article made by the process of the invention.

### DETAILED DESCRIPTION OF THE INVENTION

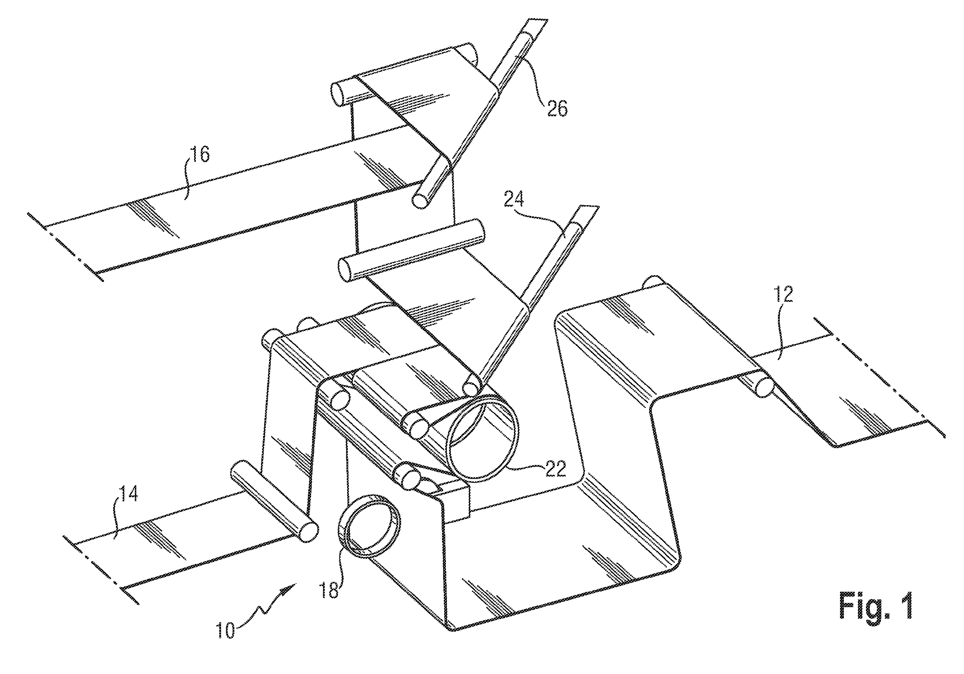

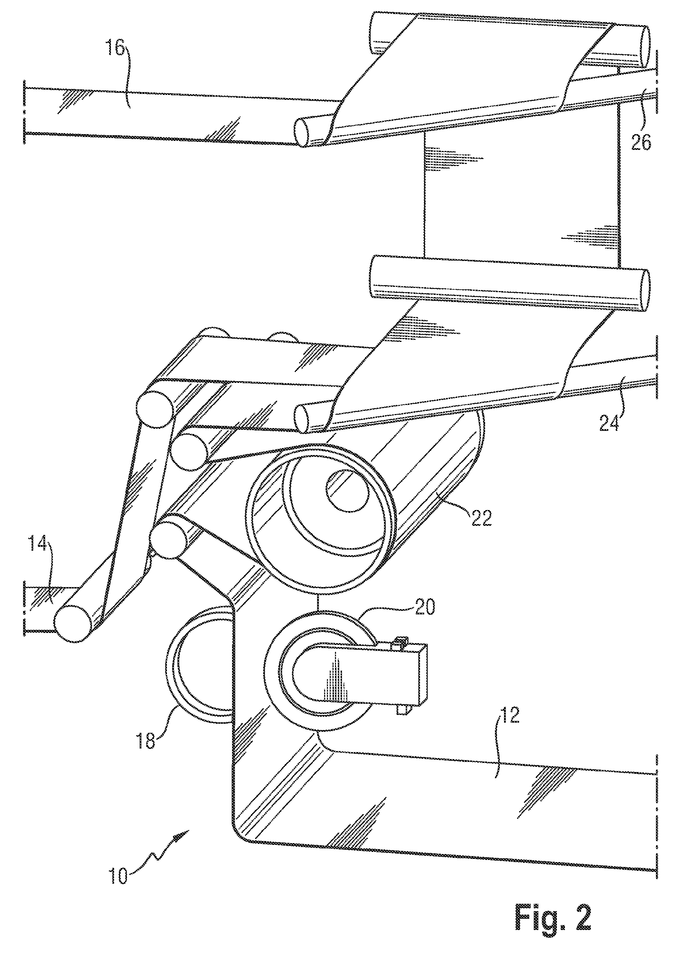

The present invention may be described by starting with the schematic representation of a slitting device 10 shown in FIGS. 1 and 2 which is part of the converting line of the invention. A nonwoven web 12 is continuously fed into the device as input. The nonwoven web 12 may be provided by unwinding a roll of material, as is usually provided by a nonwoven material supplier. The output of the device is a first nonwoven sub-web 14 and second nonwoven sub-web 16 of approximately equal width which are used further in the process to make the absorbent article.

As used herein, "approximately equal width" means that the difference in width between the first and second sub-webs is less than about 40%, preferably less than about 30%, more preferably less than about 20%, and even more preferably less than about 10%, based on the width of the widest of the first or second sub-web.

As will be described in further details hereinafter, the first sub-web 14 may be combined later with the apertured formed film to form a composite topsheet, whereas the second sub-web may be used to serve as a secondary topsheet, placed between the topsheet (e.g. a composite topsheet) and the absorbent core. The denomination "first" and "second" is of course entirely subjective as the first and second sub-webs are essentially similar materials as they exit the slitting device and the denomination "first" and "second" may therefore be used interchangeably.

The slitting device 10 as shown in FIGS. 1 and 2 comprises a standard blade 18 and anvil 20 system for cutting the nonwoven web approximately along its middle. A driving roll 22 may be placed after the blade and anvil system to keep the web well-tensed during the slitting step and to help separate the slitted nonwoven web into first and second sub-webs. The position of the blade 18 and anvil 20 is interchangeable.

Advantageously one of the sub-webs (the first sub-web 14 in the Figures, but it may be the second sub-web 16) is positioned so that it exits the slitting device already generally aligned with the other materials and the converting line so that the first sub-web does not need realigning. The second sub-web 16 then of course exits the slitting device in an offset position respective to the first sub-web 14 and the converting line so that it needs to be realigned with the other materials. This may be advantageously done by using two angled bars 24, 26 to realign the second sub-web 16 with the first sub-web 14 and the other materials in the converting line. The two angled bars 24, 26 are preferably each angled at about 45 degrees relative to the machine direction of the nonwoven web.

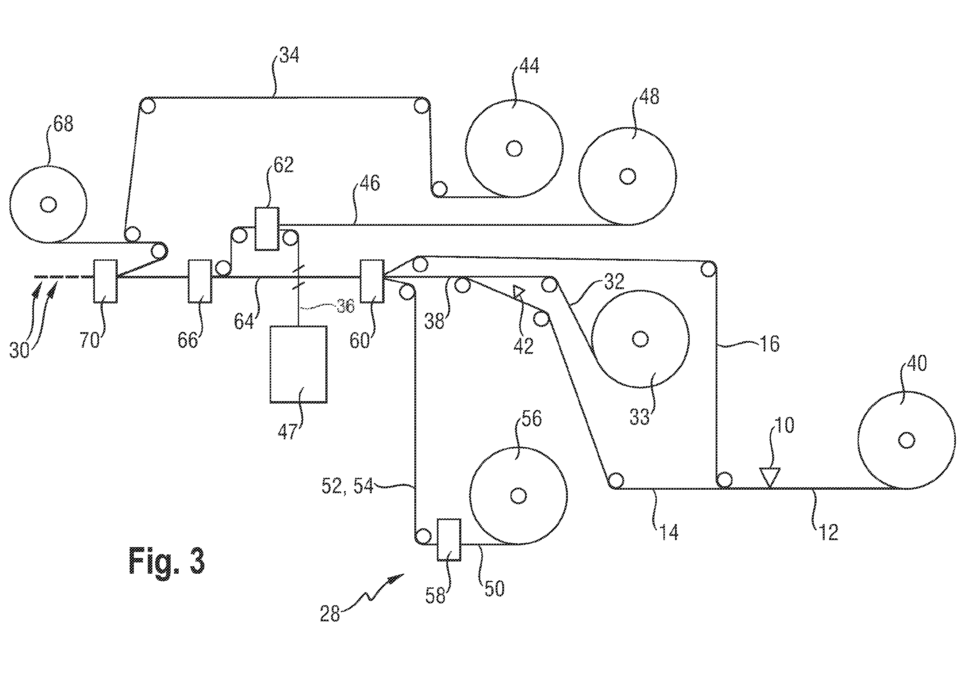

The first sub-web 14 and second sub-web 16 are then converted with other materials that are provided to the converting line 28, an embodiment of which is represented schematically in FIG. 3, for making the absorbent articles 30. The other materials comprise at least an apertured formed film 32, a backsheet material 34 and an absorbent core material 36.

The present process was found to be particularly useful wherein the different materials are converted such that the first sub-web 14, the apertured formed film 32, the second sub-web 16, the absorbent core material 36 and the backsheet material 34 are superposed, in that order or the reverse order, and the absorbent articles 30 being then formed by attaching and cutting the materials together. As indicated above, the absorbent article may of course comprise further materials placed in any positions if additional functions are sought.

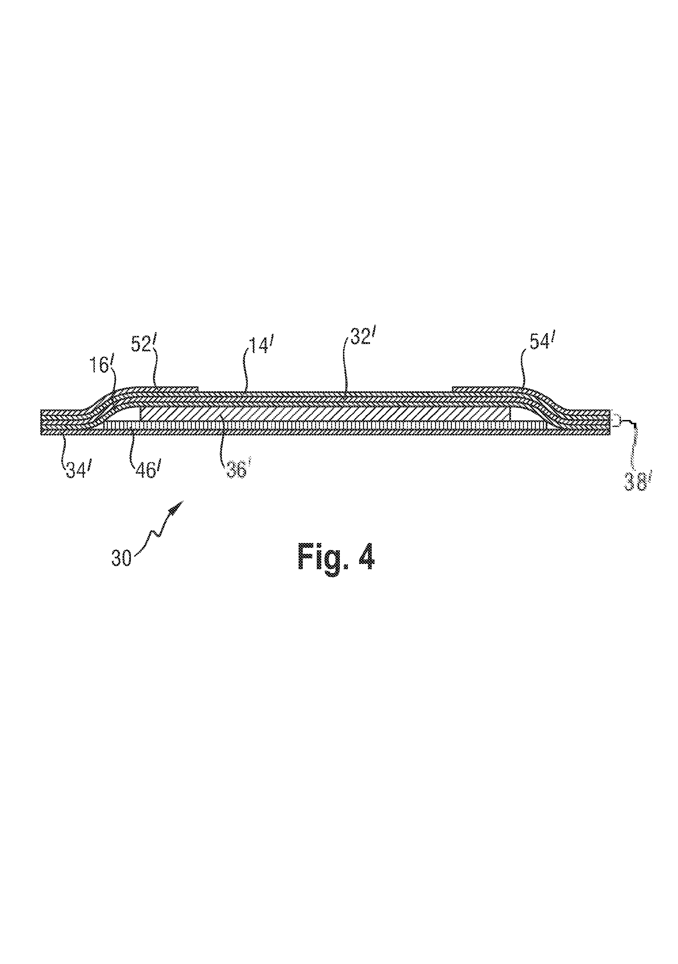

Thus, with the present process, an absorbent article 30 (an example of which is shown in cross-section in FIG. 4) is provided wherein a single roll of nonwoven material 12 can be processed in a way to serve as both a top layer 14' of a composite topsheet 38 and as a secondary topsheet 16'. It is surprisingly found that the same original nonwoven material 12 may be used to provide different functions at different locations within the absorbent articles. Such an article combines a soft touch surface provided by the nonwoven top layer 14' of the topsheet with rapid acquisition and distribution of bodily fluids (such as urine and/or menses) provided by the formed film 32' and the nonwoven secondary topsheet 16'. The present process provides a cost effective way of making such an article in comparison to other processes. In particular, the continuous splitting of the nonwoven material into two sub-webs eliminates the need of an additional unwinder, reduces waste due to material handling (caused by the need for splicing when changing rolls of materials), and reduces line layout floor space in a manufacturing plant. In comparison, a splitting device as used in the present process is comparatively low cost and takes up little space.

FIG. 3 shows an exemplary converting line employing the process of the invention. The converting line 28 comprises a roll 40 of nonwoven material 12 which is used to feed the slitting device 10 as described previously. The first sub-web 14 exiting the slitting device is directed to a first gluing station 42 where a layer of glue is applied and the first sub-web 14 is then attached to the formed film 32 (unrolled from roll 33) to form a composite topsheet material 38 comprising a top layer made of the first non-woven sub-web 12 and a bottom layer made of an apertured formed film 32. The terms "bottom" and "top" refers to the direction of the finished article 30 as it is used by the wearer, that it is the nonwoven layer 14' of the topsheet is facing the user during use, although in the exemplified converting line the absorbent article is assembled upside down, so that the top layer of the article is actually facing down in the converting line. Of course the converting line may also be set up so that the materials would be superposed in the reverse order.

In the converting line 28 exemplary described, the backsheet material 34 is provided by unrolling a continuous roll 44 of backsheet material. The absorbent core material 36 can be provided from a conventional core bale 47, which can be described as a continuous band of material that is stored flat in a quadratic container. In addition to the above described materials, other materials may be provided to the converting line to provide additional layers to the finished absorbent articles. As exemplarily shown in FIG. 3, a secondary backsheet material 46 for example in the form of a formed film, may be provided from a roll 48 of such material. A nonwoven material 50 which is continuously cut into two stripes 52, 54 in a cutting station 58 may be also provided from a roll 56 to form lateral topsheet stripes 52', 54', such as those described in WO 2007/116347 A2.

As exemplarily shown in FIG. 3, the composite topsheet 38 formed by attaching the first sub-web 14 and the apertured formed film 32 is combined with the second sub-web 16 on the film side (e.g. the second sub-web 16 serving as a secondary topsheet), and with the lateral topsheet stripes 52, 54 superposed on the first sub-web 14 at a combining unit 60. In parallel, the secondary backsheet 46 and the absorbent core material 36 can be attached together at a gluing station 62 and then combined with the lateral topsheet stripes / composite topsheet / secondary topsheet combination 64. These layers may be attached together by crimping the layers along the perimeter of the absorbent article to be made and/or gluing at a crimping and gluing station 66. The backsheet material 34 and the release paper material 68 may be, as represented, be attached by gluing to the other materials. The article 30 itself is then made by cutting with a knife the superposed and attached materials at a cutting station 70 before being sent to a filling and packaging line (not represented).

FIG. 4 shows a cross-section of an exemplary absorbent article 30 (glue not represented), comprising lateral topsheet stripes 52', 54', a composite topsheet 38' comprising a nonwoven top layer 14' and an apertured film bottom layer 32', an absorbent core 48', a secondary backsheet 46', and a backsheet 34'. Glue (not represented) may be applied between any of the layers to provide bonding and on the external surface of the backsheet for attachment of the article on the wearer's undergarment. The absorbent article may of course also comprise a wrapper material or release paper (not represented) as is usual.

The nonwoven material 12 that is used to make the first sub-web 14 and the second sub-web 16 may be made of any suitable conventional nonwoven materials, such as carded thermal bonded, spun bonded, hydro entangled, melt blown, and using all range of suitable synthetic or natural fibers such as polypropylene, polyethylene, polyester, rayon, cotton, and in a mixed form or in the form of monocomponent, bicomponent fiber. For example, Pegas a.s (Czech Republic) supplies a suitable nonwoven material comprised of bicomponent fibers having a polypropylene (PP) core and a polyethylene (PE) sheath, with a polymer ratio of PP core 70% / PE sheath 30%. The basis weight of the nonwoven material can generally be from about 10 to about 40 grams per square meter ("gsm"), preferably from about 10 to about 20 gsm.

The formed film 32 may be any apertured formed film suitable for use as a topsheet individually or as part of a composite topsheet. Suitable apertured formed films and methods for making them are described in US 4,629,643, US 4,839,216, US 6,599,612, US 6,852,475, US 7,029,264, and US 7,201,853.

The other components such as the absorbent core material 36, the secondary backsheet 46, the backsheet material 34, and the lateral topsheets stripe material 50 can be made of any conventional suitable materials usually used for this purpose. Suitable materials are described, for example, in WO 2007/116347 A2.

## Claims

1. A continuous process for making absorbent articles (30) in a converting line (28), said process comprising the step of providing a nonwoven web (12), an apertured formed film (32), a backsheet material (34), and an absorbent core material (36);
**characterized in that** said process further comprises the steps of:
slitting the nonwoven web (12) along its length to form a first sub-web (14) and a second sub-web (16) of approximately equal widths;
superposing the first sub-web (14), the apertured formed film (32), the second sub-web (16), the absorbent core material (36), and the backsheet material (34) **in that** order or reverse order; and
forming the absorbent article (30) by attaching and cutting the superposed first sub-web(14), the apertured formed film (32), the second sub-web (16), the absorbent core material (36), and the backsheet material (34).

2. A process according to Claim 1, wherein the first sub-web (14) is attached to the apertured formed film (32) to form a composite topsheet material (38) before being attached to the other materials.

3. A process according to any of the preceding claims, wherein the first sub-web (14) is glued to the apertured formed film (32).

4. A process according to any of the preceding claims, wherein the step of slitting the nonwoven web (12) comprises the steps of:
providing a slitting device (10) comprising a blade (18), an anvil (20), and a drive roll (22), and
continuously feeding the nonwoven web (12) into the slitting device (10) to cut and separate the nonwoven web (12) into the first sub-web (14) and second sub-web (16).

5. A process according to Claim 4, wherein the slitting device (10) further comprises two angled bars (24, 26).

6. A process according to any of the preceding claims, wherein the first sub-web or the second sub-web is initially offset versus the other materials and is centered with the other materials using two angled bars (24, 26).

7. A process according to any of Claims 5 or 6, wherein the two angled bars (24, 26) are each angled at 45 degrees relative to a machine direction of the nonwoven web.

8. A process according to any of the preceding claims, wherein the superposed materials are at least partially attached by crimping along the periphery of the absorbent article and then cutting along this periphery to form the absorbent article.

9. A process according to any of the preceding claims, wherein the process further comprises the step of superposing a secondary backsheet (46) with respect to the backsheet material (34) or the absorbent core material (36).

10. A process according to any of the preceding claims, wherein the nonwoven web (12) has a basis weight of from 10 to 40 grams per square meter, preferably from 10 to 20 grams per square meter.

11. A process according to any of the preceding claims, wherein the nonwoven web (12) comprises bicomponent fibers.

12. A process according to any of the preceding claims, wherein the absorbent articles (30) are feminine hygiene products.

## Patentansprüche

1. Kontinuierliches Verfahren zur Herstellung von Absorptionsartikeln (30) in einer Konvertierungslinie (28), wobei das Verfahren den Schritt der Bereitstellung einer Vliesbahn (12), einer mit Öffnungen versehenen geformten Folie (32), eines Unterschichtmaterials (34) und eines Absorptionskernmaterials (36) umfasst;
**dadurch gekennzeichnet, dass** das Verfahren ferner die folgenden Schritte umfasst:
Schlitzen der Vliesbahn (12) entlang ihrer Länge, um eine erste Teilbahn (14) und eine zweite Teilbahn (16) von ungefähr gleicher Breite zu bilden;
Übereinanderlegen der ersten Teilbahn (14), der mit Öffnungen versehenen geformten Folie (32), der zweiten Teilbahn (16), des Absorptionskernmaterials (36) und des Unterschichtmaterials (34) in dieser Reihenfolge oder in umgekehrter Reihenfolge; und
Bilden des Absorptionsartikels (30) durch Verbinden und Schneiden der übereinandergelegten ersten Teilbahn (14), der mit Öffnungen versehenen geformten Folie (32), der zweiten Teilbahn (16), des Absorptionskernmaterials (36) und des Unterschichtmaterials (34).

2. Verfahren nach Anspruch 1, wobei die erste Teilbahn (14) mit der mit Öffnungen versehenen geformten Folie (32) verbunden ist, um ein Verbund-Oberschichtmaterial (38) zu bilden, bevor sie mit den anderen Materialien verbunden wird.

3. Verfahren nach einem der vorstehenden Ansprüche, wobei die erste Teilbahn (14) an die mit Öffnungen versehene geformte Folie (32) geklebt ist.

4. Verfahren nach einem der vorstehenden Ansprüche, wobei der Schritt des Schlitzens der Vliesbahn (12) die folgenden Schritte umfasst:
Bereitstellen einer Schlitzvorrichtung (10), umfassend eine Klinge (18), einen Amboss (20) und eine Antriebswalze (22), und
kontinuierliches Zuführen der Vliesbahn (12) in die Schlitzvorrichtung (10), um die Vliesbahn (12) in die erste Teilbahn (14) und die zweite Teilbahn (16) zu schneiden und zu trennen.

5. Verfahren nach Anspruch 4, wobei die Schlitzvorrichtung (10) ferner zwei abgewinkelte Stangen (24, 26) umfasst.

6. Verfahren nach einem der vorstehenden Ansprüche, wobei die erste Teilbahn oder die zweite Teilbahn mit Hilfe von zwei abgewinkelten Stangen (24, 26) anfänglich gegenüber den anderen Materialien versetzt wird und mit den anderen Materialien zentriert wird.

7. Verfahren nach einem der Ansprüche 5 oder 6, wobei die zwei abgewinkelten Stangen (24, 26) jeweils um 45 Grad relativ zu einer Maschinenlaufrichtung der Vliesbahn abgewinkelt sind.

8. Verfahren nach einem der vorstehenden Ansprüche, wobei die übereinandergelegten Materialien wenigstens teilweise durch Kräuseln entlang dem Umfang des Absorptionsartikels und anschließendes Schneiden entlang diesem Umfang verbunden werden, um den Absorptionsartikel zu bilden.

9. Verfahren nach einem der vorstehenden Ansprüche, wobei das Verfahren ferner den Schritt des Übereinanderlegens einer sekundären Unterschicht (46) in Bezug auf das Unterschichtmaterial (34) oder das Absorptionskernmaterial (36) umfasst.

10. Verfahren nach einem der vorstehenden Ansprüche, wobei die Vliesbahn (12) ein Basisgewicht von 10 bis 40 Gramm pro Quadratmeter, vorzugsweise von 10 bis 20 Gramm pro Quadratmeter, besitzt.

11. Verfahren nach einem der vorstehenden Ansprüche, wobei die Vliesbahn (12) Bikomponentenfasern umfasst.

12. Verfahren nach einem der vorstehenden Ansprüche, wobei die Absorptionsartikel (30) Damenhygieneprodukte sind.

## Revendications

1. Procédé continu pour fabriquer des articles absorbants (30) dans une chaîne de transformation (28), ledit procédé comprenant l'étape consistant à fournir une nappe non tissée (12), une pellicule formée perforée (32), un matériau de feuille de fond (34) et un matériau d'âme absorbante (36) ;
**caractérisé en ce que** ledit procédé comprend en outre les étapes consistant à :
refendre la nappe non tissée (12) sur toute sa longueur pour former une première sous-nappe (14) et une deuxième sous-nappe (16) de largeurs approximativement égales ;
superposer la première sous-nappe (14), la pellicule formée perforée (32), la deuxième sous-nappe (16), le matériau d'âme absorbante (36) et le matériau de feuille de fond (34) dans cet ordre ou dans l'ordre inverse ; et
former l'article absorbant (30) en fixant et en coupant la première sous-nappe superposée (14), la pellicule formée perforée (32), la deuxième sous-nappe (16), le matériau d'âme absorbante (36) et le matériau de feuille de fond (34).

2. Procédé selon la revendication 1 dans lequel la première sous-nappe (14) est fixée à la pellicule formée perforée (32) pour former un matériau de feuille de dessus composite (38) avant d'être fixée aux autres matériaux.

3. Procédé selon l'une quelconque des revendications précédentes dans lequel la première sous-nappe (14) est collée à la pellicule formée perforée (32).

4. Procédé selon l'une quelconque des revendications précédentes dans lequel l'étape consistant à refendre la nappe non tissée (12) comprend les étapes consistant à :
fournir un dispositif de refendage (10) comprenant une lame (18), une enclume (20) et un rouleau d'entraînement (22), et
alimenter en continu la nappe non tissée (12) dans le dispositif de refendage (10) pour couper et séparer la nappe non tissée (12) en la première sous-nappe (14) et la deuxième sous-nappe (16).

5. Procédé selon la revendication 4 dans lequel le dispositif de refendage (10) comprend en outre deux barres inclinées (24, 26).

6. Procédé selon l'une quelconque des revendications précédentes dans lequel la première sous-nappe ou la deuxième sous-nappe est initialement décalée par rapport aux autres matériaux et est centrée avec les autres matériaux à l'aide de deux barres inclinées (24, 26).

7. Procédé selon l'une quelconque des revendications 5 ou 6 dans lequel les deux barres inclinées (24, 26) sont chacune inclinée de 45° par rapport à un sens machine de la nappe non tissée.

8. Procédé selon l'une quelconque des revendications précédentes dans lequel les matériaux superposés sont au moins partiellement fixés par sertissage le long de la périphérie de l'article absorbant, puis par découpe le long de cette périphérie pour former l'article absorbant.

9. Procédé selon l'une quelconque des revendications précédentes dans lequel le procédé comprend en outre l'étape consistant à superposer une feuille de fond secondaire (46) sur le matériau de feuille de fond (34) ou le matériau d'âme absorbante (36).

10. Procédé selon l'une quelconque des revendications précédentes dans lequel la nappe non tissée (12) a une masse surfacique comprise entre 10 et 40 grammes par mètre carré, de préférence entre 10 et 20 grammes par mètre carré.

11. Procédé selon l'une quelconque des revendications précédentes dans lequel la nappe non tissée (12) comprend des fibres bicomposants.

12. Procédé selon l'une quelconque des revendications précédentes dans lequel les articles absorbants (30) sont des produits d'hygiène féminine.
